# EUROPEAN PATENT SPECIFICATION

(11) **EP 3 061 428 B1**
(45) Date of publication and mention of the grant of the patent: **10.04.2019**
(21) Application number: 16156644.3
(22) Date of filing: 22.02.2016
(51) Int. Cl.: A61F 5/37

(54) **ORTHOPAEDIC SLING WITH REMOVABLE FABRIC COATINGS**
ORTHOPÄDISCHE SCHLINGE MIT ABNEHMBAREM STOFFÜBERZUG
ECHARPE ORTHOPEDIQUE COMPORTANT UN REVETEMENT DETACHABLE DE TISSU

(30) Priority: 26.02.2015 IT RM20150086
(43) Date of publication of application: 31.08.2016
(73) Proprietor: Moneta, Maria Rita, 00136 Rome (IT)
(72) Inventor: Moneta, Maria Rita, 00136 Rome (IT)
(74) Representative: Scilletta, Andrea

(56) References cited:
- US-A1- 2008 228 116
- US-A1- 2010 121 237
- US-A1- 2011 192 403
- US-A1- 2011 213 282

## Description

The present invention generally relates to an orthopaedic sling, configured to support an upper limb of a user, provided with one or more removable fabric coatings, optionally washable, more optionally breathable, that allows in a reliably, comfortable and inexpensive manner to keep the sling clean. The sling according to the invention can further allow to change its appearance according to the user's requirements.

There are many different orthopaedic slings on the market, configured to support and/or immobilise an upper limb of a user, having specific features depending on different required functionalities, such as immobilisation and support, blocking a segment, directional control of the movement, facilitation of the limb functions for treatments such as immobilisation of the elbow close to the body after traumatic events of the shoulder and elbow, immobilisation of the shoulder after dislocation and/or surgery (for blocking the external rotation), support of the shoulder after hemiplegia or brachial plexus injury.

Several prior art documents disclose various solutions proposed as orthopedic slings for the upper limb. A universal harness for upper limb and shoulder immobiliser is disclosed in prior art document US4372301, wherein the immobiliser is made of flexible material. Document US5334132 (A) discloses an improved arm sling that includes a forearm support section having an opening to enable the user, possibly with the aid of a doctor or therapist, to perform exercises, to flex and extend the forearm. Document US2013317401 discloses an assembly for supporting a patient's arm, including a waist band, a pillow and an arm harness, wherein these elements are provided with coupling means in order to be coupled to each other. Document EP1013248 discloses an immobiliser device for shoulder and upper limb, consisting of a thoracic strap and a forearm support for the treatment of trauma, dislocations, sprains or surgery of upper limb and shoulder.

Other prior art slings are disclosed in documents US 2010/0121237 A1, which discloses an orthopaedic sling in accordance with the preamble of claim 1, US 2008/0228116 A1 and US2011/0213282 A1.

The prior art slings suffer from the drawback of being commonly constituted of synthetic material, and since a similar sling must be worn for a period that can range from a few days to several weeks during which the user can not remove it, the sling easily gets dirty and cannot be efficiently washed to the detriment of hygiene. This implies that, in case of dirt or damage of the outer surfaces of the sling, in order to restore the initial state of the sling, it is necessary to remove it and properly clean it and/or entirely replace it with another sling. In this regard, the operations for an efficient cleaning (such as, for instance, washing) can damage some structural and/or non-washable parts of the sling, such as for instance the coupling parts of the harnesses, foam pillows for limb abduction; in the case where a replacement is required, there is an increase of costs for the user. A further drawback is that, if the need of replacing the sling occurs during its use by the user, the possible damage of structural parts and/or the replacement operations may induce incorrect movements of the limb supported by the sling affecting the recovery of limb functionality.

Therefore, it is an object of the present invention is to overcome the drawbacks described hereinbefore, allowing in a reliable, comfortable and inexpensive way to keep the sling clean.

It is specific subject-matter of the present invention to describe an orthopaedic sling as claimed in independent claim 1.

Further embodiments of the invention are claimed in dependent claims.

In other words, the orthopaedic sling according to the invention comprises a structure having a plurality of elements that is configured to support an upper limb of a user, wherein the orthopaedic sling is provided with at least one removable fabric coating, optionally washable, more optionally breathable, configured to be removably coupled to at least one component of the structure.

The present invention allows to obtain the following main advantages compared to the solutions present in the state of the art. The main advantage of the present invention is to keep an upper limb sling intact and clean, allowing to efficiently wash and/or replace only the removable fabric coating, optionally washable, more optionally breathable, that is dirty and/or damaged, consequently improving the hygiene and reducing the costs for the user.

A further advantage is that the aforementioned operations for restoring the initial conditions of the sling may occur during its use by the patient without inducing incorrect movements of the engaged limb, thus ensuring the full and continuous functionality of the sling. Another advantage is that the removable fabric coating can be removably coupled to the components of the structure in a simple way even by the same user, adapting to the possibly reduced mobility of the user. The present invention further allows to reduce erythema and/or decubitus of the skin caused by components of the sling structure which get in contact with the user's skin.

The present invention will be now described, by way of illustration and not by way of limitation, according to a preferred embodiment, by particularly referring to the drawings of the annexed Figures, in which:
Figure 1 shows a front perspective view of a user wearing a structure of a preferred embodiment of the orthopaedic sling according to the invention;
Figure 2 shows a front perspective view of a user wearing the orthopaedic sling having the structure of Figure 1.

In the Figures, identical reference numerals will be used for alike elements.

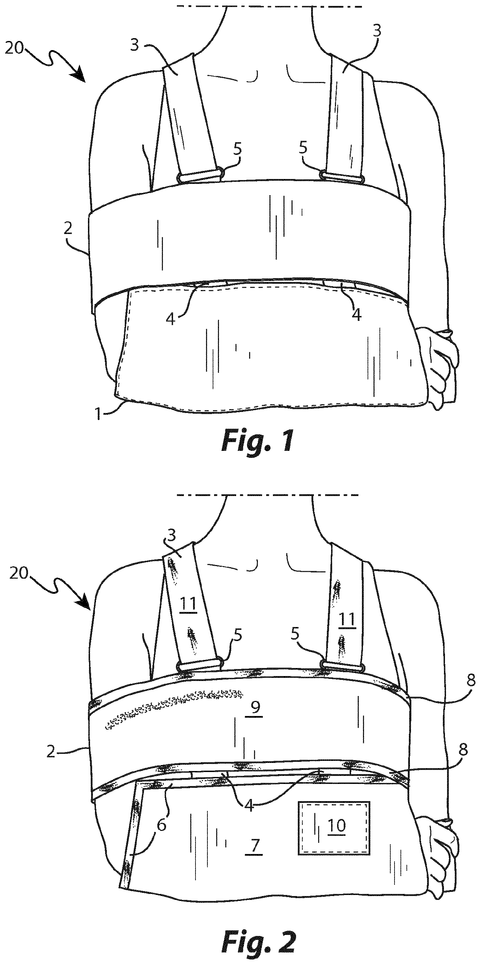

In Figure 1, it is shown the structure of the preferred embodiment of the orthopaedic sling according to the invention, which structure includes a plurality of components, namely: a sleeve 1, operating as a containing pocket configured to contain and support the right forearm of the user 20, two suspension straps 3 (the rear portions of which, configured to be worn on the back of the user 20, can be optionally configured to be crossed), and an immobiliser chest band 2. The front portions, configured to be worn on the chest of the user 20, of the two suspension straps 3 can be optionally coupled, more optionally in a removable manner, even more optionally in an adjustable manner, to the rear portions of the two suspension straps 3 through first mechanical coupling means (not shown), such as self-locking buckle closures and/or zippers and/or buttons and buttonholes and/or magnetic buttons and/or Austrian knots and/or clips and/or hook and loop fabric fastening devices (also known with the trade name Velcro®). The rear portion, configured to be worn on the back of the user 20, of the chest band 2 can be optionally coupled to the rear portions of the suspension straps 3. The sleeve 1 is supported by the front portion, configured to be worn on the chest of the user 20, of the two suspension straps 3; to this end, the sleeve 1 is directly coupled, optionally in a removable manner, to the front portions of the two suspension straps 3 through second mechanical coupling means 4, optionally adjustable, more optionally removable, such as self-locking buckle closures and/or zippers and/or buttons and buttonholes and/or magnetic buttons and/or Austrian knots and/or clips and/or hooks and/or hook and loop fabric fastening devices. Other embodiments of the orthopaedic sling according to the invention may comprise, alternatively to or in combination with the second mechanical coupling means 4, one or more loops coupled, optionally in a removable manner, to the front portions of the two suspension straps 3 into which the sleeve 1 can be inserted. Optionally, also the front portion, configured to be worn on the chest of the user 20, of the immobiliser chest band 2 can be coupled to the front portion, more optionally in correspondence of the end of the front portion, of at least one of the suspension straps 3 through third coupling means 5, optionally adjustable, more optionally removable, such as self-locking buckle closures and/or zippers and/or buttons and buttonholes and/or magnetic buttons and/or Austrian knots and/or clips and/or hooks and/or hook and loop fabric fastening devices. In particular, the immobiliser chest band 2 can be optionally made of elastic material.

As shown in Figure 1, during the use of the orthopaedic sling, the right forearm of a user 20 is inserted into the sleeve 1, the suspension straps 3 are passed on the shoulders of the user 20, so that the second and third mechanical coupling means 4 and 5 are on the chest of the user 20, and the rear portions of the straps 3, optionally crossed, are on the back of the user 20. Optionally, the height of the sleeve 1 with respect to the chest of the user 20 can be adjusted through the second mechanical coupling means 4, when these are adjustable, and also through the possibly adjustable first coupling means. Finally, the immobiliser chest band 2 is passed around the torso of the user 20 in a complete loop, leaving the elbow and hand free, until the chest band 2 is fastened to itself in an adjustable manner, for example through hook and loop fastening fabric devices and/or through ropes and hooks and/or through clips with adjustable length bands.

In Figure 2, the complete orthopaedic sling, having the structure of Figure 1, is shown. The sleeve 1 includes on its outer surface and on its inner surface, i.e. the surface facing the arm of the user 20, first removable coupling elements (not shown) interacting with second removable coupling elements 6 with which one or more first fabric coatings, optionally washable, more optionally breathable, are provided, each one of which is thus configured to be removably coupled, through the interaction of the first and second removable coupling elements 6, to at least one portion of the inner surface and/or of the outer surface of the sleeve 1 (one of such first coatings is visible in Figure 2, indicated with the reference numeral 7, coupled to the outer surface of the sleeve 1). Preferably, the first and the second removable coupling elements 6 are part of hook and loop fastening fabric devices; alternatively to or in combination with the hook and loop fastening fabric devices, the first and second removable coupling elements 6 may comprise self-locking buckle closures and/or zippers and/or buttons and buttonholes and/or magnetic buttons and/or Austrian knots and/or clips and/or hooks. Advantageously, said one or more first coatings substantially entirely cover both the inner surface and the outer surface of the sleeve 1. In the preferred embodiment of the orthopaedic sling according to the invention shown in the Figures, there are two first coatings which are respectively fastened to the outer surface and to the inner surface of the sleeve 1, covering them entirely, whereby such first coatings are properly shaped so as to have a shape corresponding to the shape of the inner surface and of the outer surface, respectively, of the sleeve to which they are coupled. Moreover, the first coating 7 coupled to the outer surface of the sleeve 1 is provided with a pocket 10, usable for storing objects, such as for instance pillboxes, blister packs, boxes of drugs, handkerchiefs, wallets, mobile phones, and portable radios, which are easily accessible to the user 20 even through the left hand, the movements of which are not constrained by the orthopaedic sling; advantageously, the pocket 10 is arranged on a portion of the first coating 7 that is directed toward the outside, i.e. not towards the sleeve 1. It should be understood that other embodiments of the orthopaedic sling according to the invention may comprise a first coating configured to fold and couple to both the outer surface and the inner surface of the sleeve 1 to cover them entirely, and/or they may include a number of first coatings larger than two, each one of which entirely or partially covers the outer surface and/or the outer surface of the sleeve 1. Moreover, other embodiments of the orthopaedic sling according to the invention can be devoid of the pocket 10, or they may include more than one pocket accessible to the user 20.

Similarly, each one of the two suspension straps 3 includes on its outer surface and on its inner surface, i.e. on the surface facing the chest of user 20, third removable coupling elements (not shown) interacting with fourth removable coupling elements (not shown) with which one or more second fabric coatings, optionally washable, more optionally breathable, are provided, each one of which is thus configured to be removably coupled, through the interaction of the third and fourth removable coupling elements, to at least one portion of the inner surface and/or the outer surface of the respective suspension strap 3 (two second coatings 11 each coupled to the outer surface of a respective suspension strap 3 are partially visible in Figure 2). Preferably, the third and the forth removable coupling elements may include self-locking buckle closures and/or zippers and/or buttons and buttonholes and/or magnetic buttons and/or Austrian knots and/or clips and/or hooks. Advantageously, said one or more second coatings substantially entirely cover both the inner surface and the outer surface of each one of the two suspension straps 3. In the preferred embodiment of the orthopaedic sling according to the invention shown in the Figures, there are two second coatings each one of which is configured to fold and coupled to a respective suspension strap 3 for entirely covering both the outer surface and the inner surface thereof. However, it should be understood that other embodiments of the orthopaedic sling according to the invention may comprise a number of second coatings larger than two, each one of which entirely or partially covers the outer surface and/or the outer surface of a respective suspension strap 3. Furthermore, other embodiments of the orthopaedic sling according to the invention may comprise at least one second coating provided with at least one pocket, similar to the pocket 10 of the first coating 7, which is easily accessible to the user 20 also through the left hand, the movements of which are not constrained by the orthopaedic sling.

In the orthopaedic sling shown in the Figures, the chest band 2 includes on its outer surface and on its inner surface, i.e. the surface facing the chest of the user 20, fifth removable coupling elements (not shown) interacting with sixth removable coupling elements 8 with which one or more third fabric coatings, optionally washable, more optionally breathable, are provided, each one of which is thus configured to be removably coupled, through the interaction of the fifth and sixth removable coupling elements 8, to at least one portion of the inner surface and/or the outer surface of the chest band 2 (a third coating 9 coupled to the outer surface of the chest band 2 is visible in Figure 2). Preferably, the fifth and the sixth removable coupling elements 8 are part of hook and loop fabric fastening devices; alternatively to or in combination with the hook and loop fabric fastening devices, the fifth and the sixth removable coupling elements 8 may include self-locking buckle closures and/or zippers and/or buttons and buttonholes and/or magnetic buttons and/or Austrian knots and/or clips and/or hooks. Advantageously, said one or more third coatings substantially entirely cover both the inner surface and the outer surface of the chest band 2. In the preferred embodiment of the orthopaedic sling according to the invention shown in the Figures, there are two third coatings which are respectively fastened to the outer surface and the inner surface of the chest band 2 covering them entirely, whereby such third coatings are properly shaped so as to have a shape corresponding to the shape of the inner surface and the outer surface, respectively, of the chest band 2 to which they are coupled (leaving the fastening means of the chest band 2 exposed and free to interact on itself). However, it should be understood that other embodiments of the orthopaedic sling according to the invention may include a third coating configured to fold and couple to both the outer surface and the inner surface of the chest band 2, and/or they may include a number of third coatings larger than two, each one of which entirely or partially covers the outer surface and/or the outer surface of the chest band 2.

Moreover, other embodiments of the orthopaedic sling according to the invention may comprise at least one third coating provided with at least one pocket, similar to the pocket 10 of the first coating 7, that is easily accessible to the user 20 also through the left hand, the movements of which are not constrained by the orthopaedic sling.

Further embodiments of the orthopaedic sling according to the invention may have structures different from that shown in Figure 1 for the preferred embodiment, for instance devoid of the chest band 2 and/or comprising a single suspension strap 3 to and/or including further components of the structure, such as for instance pillows for shoulder abduction. In addition, although the preferred embodiment of the sling according to the invention comprises a sleeve 1 configured to contain and support the right forearm of a user 20, other embodiments of the sling according to the invention may have a structure comprising a sleeve configured to contain and support the left forearm of a user, and further embodiments of the sling according to the invention may have a structure comprising at least one sleeve configured to contain and support one or more portions of varying length of an upper, either right or left, limb of a user (for example, a wrist and/or a hand and/or an elbow).

The preferred embodiments of this invention have been described and a number of variations have been suggested hereinbefore, but it should be understood that those skilled in the art can make other variations and changes, without so departing from the scope of protection thereof, as defined by the enclosed claims.

## Claims

1. Orthopaedic sling, configured to support an upper limb of a user (20), comprising a structure including at least one sleeve (1), configured to contain and support portions of an upper limb of the user (20), one or more first fabric coatings (7), and at least one suspension strap (3), wherein said at least one sleeve (1) is coupled to said at least one suspension strap (3) through coupling means (4), wherein said at least one sleeve (1) includes first coupling elements interacting with second coupling elements (6) with which said one or more first fabric coatings (7) are provided, whereby each one of said one or more first fabric coatings (7) is configured to be removably coupled, through the interaction of the first and second coupling elements (6), to at least one portion of a surface of said at least one sleeve (1), **characterised in that** said at least one suspension strap (3) includes third coupling elements interacting with fourth coupling elements with which one or more second fabric coatings (11) are provided, whereby each one of said one or more second fabric coatings (11) is configured to be removably coupled, through the interaction of the third and fourth coupling elements, to at least one portion of said at least one suspension strap (3), wherein the third and fourth coupling elements comprise self-locking buckle closures and/or zippers and/or buttons and buttonholes and/or magnetic buttons and/or Austrian knots and/or clips and/or hooks.

2. Orthopaedic sling according to claim 1, **characterised in that** the first and second coupling elements (6) are part of hook and loop fabric fastening devices.

3. Orthopaedic sling according to claim 1 or 2, **characterised in that** the first and second coupling elements (6) comprise self-locking buckle closures and/or zippers and/or buttons and buttonholes and/or magnetic buttons and/or Austrian knots and/or clips and/or hooks.

4. Orthopaedic sling according to any one of the preceding claims, **characterised in that** at least one of said one or more first fabric coatings (7) is provided with at least one pocket (10).

5. Orthopaedic sling according to any one of the preceding claims, **characterised in that** at least one of said one or more second fabric coatings (11) is provided with at least one pocket.

6. Orthopaedic sling according to any one of the preceding claims, **characterised in that** the structure further comprises an immobiliser chest band (2) including fifth coupling elements interacting with sixth coupling elements (8) with which one or more third fabric coatings (9) are provided, whereby each one of said one or more third fabric coatings (9) is configured to be removably coupled, through the interaction of the fifth and sixth coupling elements (8), to at least one portion of a surface of the chest band (2).

7. Orthopaedic sling according to claim 6, wherein at least one of said one or more third fabric coatings (9) is provided with at least one pocket.

8. Orthopaedic sling according to claim 6 or 7, **characterised in that** the fifth and sixth coupling elements (8) are part of hook and loop fabric fastening devices.

9. Orthopaedic sling according to any one of claims 6 to 8, **characterised in that** the fifth and sixth coupling elements (8) comprise self-locking buckle closures and/or zippers and/or buttons and buttonholes and/or magnetic buttons and/or Austrian knots and/or clips and/or hooks.

## Patentansprüche

1. Orthopädische Schlinge, die dazu konfiguriert ist, eine obere Gliedmaße eines Nutzers (20) zu stützen, umfassend eine Struktur, die mindestens einen Ärmel (1) umfasst, der dazu konfiguriert ist, Teile einer oberen Gliedmaße des Nutzers (20) zu enthalten und zu stützen, einen oder mehrere erste Gewebebezüge (7), und mindestens einen Tragegurt (3), wobei der genannte mindestens eine Ärmel (1) mit dem mindestens einen Tragegurt (3) durch Kopplungsmittel (4) gekoppelt ist, wobei der mindestens eine Ärmel (1) erste Kopplungselemente umfasst, die mit zweiten Kopplungselemente (6) wechselwirken, mit denen der eine oder die mehreren ersten Gewebebezüge (7) versehen sind, wobei
ein jeder des einen oder der mehreren ersten Gewebebezüge (7) dazu konfiguriert ist, durch die Wechselwirkung der ersten und der zweiten Kopplungselemente (6) mit zumindest einem Abschnitt einer Oberfläche des mindestens einen Ärmels (1) lösbar gekoppelt zu werden, **dadurch gekennzeichnet, dass** der mindestens eine Tragegurt (3) dritte Kopplungselemente umfasst, die mit vierten Kopplungselementen wechselwirken, mit denen ein oder mehrere zweite Gewebebezüge (11) versehen sind, wobei ein jeder des einen oder der mehreren zweiten Gewebebezüge (11) dazu konfiguriert ist, durch die Wechselwirkung der dritten und vierten Kopplungselemente, mit mindestens einem Abschnitt des mindestens einen Tragegurts (3) lösbar gekoppelt zu werden, wobei die dritten und vierten Kopplungselemente selbstverschließende Schnallenverschlüsse und/oder Reißverschlüsse und/oder Knöpfe und Knopflöcher und/oder magnetische Knöpfe und/oder österreichische Knoten und/oder Klammern und/oder Haken umfassen.

2. Orthopädische Schlinge nach Anspruch 1, **dadurch gekennzeichnet, dass** das erste und das zweite Kopplungselement (6) Teil von einer Klettverschlussvorrichtung sind.

3. Orthopädische Schlinge nach Anspruch 1 oder 2, **dadurch gekennzeichnet, dass** das erste und das zweite Kopplungselement (6) selbstverschließende Schnallenverschlüsse und/oder Reißverschlüsse und/oder Knöpfe und Knopflöcher und/oder magnetische Knöpfe und/oder österreichische Knoten und/oder Klammern und/oder Haken umfassen.

4. Orthopädische Schlinge nach einem der vorhergehenden Ansprüche, **dadurch gekennzeichnet, dass** mindestens einer des einen oder der mehreren ersten Gewebebezüge (7) mit mindestens einer Tasche (10) versehen ist.

5. Orthopädische Schlinge nach einem der vorhergehenden Ansprüche, **dadurch gekennzeichnet, dass** mindestens einer des einen oder der mehreren zweiten Gewebebezüge (11) mit mindestens einer Tasche versehen ist.

6. Orthopädische Schlinge nach einem der vorhergehenden Ansprüche, **dadurch gekennzeichnet, dass** die Struktur ferner ein Immobilisierungs-Brustband (2) umfasst, das fünfte Kopplungselemente umfasst, die mit sechsten Kopplungselementen (8) wechselwirken, mit denen ein oder mehrere dritte Gewebebezüge (9) versehen ist bzw. sind, wobei ein jeder des einen oder der mehreren dritten Gewebebezüge (9) dazu konfiguriert ist, durch die Wechselwirkung der fünften und sechsten Kopplungselemente (8) lösbar mit zumindest einem Abschnitt einer Oberfläche des Brustbandes (2) gekoppelt zu werden.

7. Orthopädische Schlinge nach Anspruch 6, wobei mindestens einer des einen oder der mehreren dritten Gewebebezüge (9) mit mindestens einer Tasche versehen ist bzw. sind.

8. Orthopädische Schlinge nach Anspruch 6 oder 7, **dadurch gekennzeichnet, dass** die fünften und sechsten Kopplungselemente (8) Teil einer Klettverschlussvorrichtung sind.

9. Orthopädische Schlinge nach einem der Ansprüche 6 bis 8, **dadurch gekennzeichnet, dass** die fünften und sechsten Kopplungselemente (8) selbstschließende Schnallenverschlüsse und/oder Reißverschlüsse und/oder Knöpfe und Knopflöcher und/oder magnetische Knöpfe und/oder österreichische Knoten und/oder Klammern und/oder Haken umfassen.

## Revendications

1. Écharpe orthopédique, conçue pour soutenir un membre supérieur d'un utilisateur (20), comprenant une structure comprenant au moins un manchon (1), conçu pour contenir et soutenir des parties d'un membre supérieur de l'utilisateur (20), un ou plusieurs premiers revêtements de tissu (7),
et au moins une sangle de suspension (3), dans lequel ledit un manchon (1) est couplé à ladite au moins une sangle de suspension (3) à travers un moyen de couplage (4), dans laquelle ledit au moins un manchon (1) comprend des premiers éléments de couplage interagissant avec des deuxièmes éléments de couplage (6) avec lesquels ledit un ou plusieurs premiers revêtements de tissu (7) sont fournis, au moyen desquels
chacun dudit un ou plusieurs premiers revêtements de tissu (7) est conçu pour être couplé de façon détachable, à travers l'interaction du premier et du deuxième éléments de couplage (6), à au moins une partie d'une surface dudit au moins un manchon (1), **caractérisée en ce que** ladite au moins une sangle de suspension (3) comprend des troisièmes éléments de couplage interagissant avec des quatrièmes éléments de couplage avec lesquels un ou plusieurs seconds revêtements de tissu (11) sont fournis, au moyen desquels chacun dudit un ou plusieurs seconds revêtements de tissu (11) est conçu pour être couplé de façon détachable, à travers l'interaction du troisième et du quatrième élément de couplage, à au moins une partie de ladite au moins une sangle de suspension (3), dans laquelle le troisième et le quatrième éléments de couplage comprennent des fermetures de boucle autobloquante et/ou des fermetures éclair et/ou des boutons et des boutonnières et/ou des boutons magnétiques et/ou des noeuds autrichiens et/ou des pinces et/ou des crochets.

2. Écharpe orthopédique selon la revendication 1, **caractérisée en ce que** le premier et le deuxième éléments de couplage (6) font partie de dispositifs de fixation de tissu à boucles et à crochets.

3. Écharpe orthopédique selon la revendication 1 ou 2, **caractérisée en ce que** le premier et le deuxième éléments de couplage (6) comprennent des fermetures de boucle autobloquante et/ou des fermetures éclair et/ou des boutons et des boutonnières et/ou des boutons magnétiques et/ou des noeuds autrichiens et/ou des pinces et/ou des crochets.

4. Écharpe orthopédique selon l'une quelconque des revendications précédentes, **caractérisée en ce qu'**au moins un dudit un ou plusieurs premiers revêtements de tissu (7) est fourni avec au moins une poche (10).

5. Écharpe orthopédique selon l'une quelconque des revendications précédentes, **caractérisée en ce qu'**au moins un dudit un ou plusieurs seconds revêtements de tissu (11) est fourni avec au moins une poche.

6. Écharpe orthopédique selon l'une quelconque des revendications précédentes, **caractérisée en ce que** la structure comprend également une bande de poitrine immobilisatrice (2) comprenant des cinquièmes éléments de couplage interagissant avec des sixièmes éléments de couplage (8) avec lesquelles un ou plusieurs troisièmes revêtements de tissu (9) sont fournis, au moyen desquels chacun dudit un ou plusieurs troisièmes revêtements de tissu (9) est conçu pour être couplé de façon détachable, à travers l'interaction du cinquième et du sixième éléments de couplage (8), à l'au moins une partie d'une surface de la bande de poitrine (2).

7. Écharpe orthopédique selon la revendication 6, dans lequel au moins un dudit un ou plusieurs troisièmes revêtements de tissu (9) est fourni avec au moins une poche.

8. Écharpe orthopédique selon la revendication 6 ou 7, **caractérisée en ce que** le cinquième et le sixième éléments de couplage (8) font partie de dispositifs de fixation de tissu à boucles et à crochets.

9. Écharpe orthopédique selon l'une quelconque des revendications 6 à 8, **caractérisée en ce que** le cinquième et le sixième éléments de couplage (8) comprennent des fermetures de boucle autobloquante et/ou des fermetures éclair et/ou des boutons et des boutonnières et/ou des boutons magnétiques et/ou des noeuds autrichiens et/ou des pinces et/ou des crochets.
